# DEMANDE DE BREVET EUROPEEN

(11) **EP 1 382 295 A1**
(43) Date de publication de la demande: **21.01.2004**
(21) Numéro de dépôt: 03291695.9
(22) Date de dépôt: 08.07.2003
(51) Int. Cl.: A61B 5/103

(54) **Atlas comportant au moins une séquence vidéo**

(30) Priorité: 09.07.2002 FR 0208614
(71) Demandeur: L'OREAL, 75008 Paris (FR)
(72) Inventeur: Betra, Gisèle, 92600 Asnieres (FR)
(74) Mandataire: Tanty, François

(57) **Abrégé**

La présente invention concerne un atlas comportant une pluralité d'images correspondant à différentes gradations d'au moins une caractéristique de la typologie corporelle et au moins une séquence vidéo associée à l'une au moins des images de l'atlas, cette séquence comportant des images exprimant ladite caractéristique.

## Description

La présente invention concerne l'évaluation de caractéristiques de la typologie corporelle, notamment l'évaluation de l'état capillaire ou cutané, sain ou non.

Par « caractéristiques de la typologie corporelle », on entend au sens de la présente demande des caractéristiques mécaniques, morphologiques ou physiologiques.

Il est connu, pour évaluer la nature de la peau d'une personne, de comparer une image de sa peau avec des images d'un atlas correspondant respectivement à différentes natures de peau, par exemple des peaux plus ou moins sèches ou grasses.

Une telle évaluation peut être utile sur un point de vente par exemple, en vue de prescrire un produit de traitement.

La comparaison entre l'image de la peau dont on cherche à évaluer l'état et les images de l'atlas peut s'avérer difficile, notamment pour une personne peu habituée.

Il existe par conséquent un besoin pour bénéficier d'un atlas perfectionné permettant notamment d'évaluer plus précisément et/ou plus sûrement une caractéristique de la typologie corporelle, par exemple un état cutané ou capillaire.

L'invention permet notamment, selon l'un de ses aspects, de répondre à ce besoin grâce à un atlas qui peut se caractériser par le fait qu'il comporte une pluralité d'images correspondant respectivement à différentes gradations d'au moins une caractéristique de la typologie corporelle et au moins une séquence vidéo associée à l'une au moins des images de l'atlas, cette séquence comportant des images exprimant ladite caractéristique.

La visualisation de la séquence vidéo peut permettre à l'observateur de valider ou non plus facilement le choix d'une image de l'atlas.

En effet, il peut s'avérer plus aisé d'identifier certaines caractéristiques de l'image acquise par la caméra au sein d'une séquence vidéo que par comparaison avec une image de l'atlas.

En outre, l'image acquise par la caméra peut être une image qui a été figée sur l'écran après avoir déplacé la caméra sur la zone à évaluer.

La séquence d'image qui est apparue sur l'écran lors de ce déplacement peut ressembler plus ou moins à la séquence vidéo qui est associée à l'image de l'atlas sélectionnée et conforter l'opérateur dans le choix de cette image.

Dans le cas de l'évaluation de l'état capillaire, la visualisation d'une séquence vidéo permet en outre de tenir compte du fait qu'il n'est généralement pas possible de visualiser à l'écran une vue agrandie de la totalité d'un cheveu si celui-ci est long. Ainsi, la séquence vidéo peut permettre de visualiser l'état d'un cheveu depuis sa racine jusqu'à sa pointe et donc d'évaluer plus précisément l'état du cheveu en fonction de l'aspect en différents endroits.

Enfin, la visualisation d'une séquence vidéo permet, le cas échéant, à la personne évaluée de mieux comprendre ce qui caractérise un état capillaire ou cutané, par exemple.

Les images de l'atlas peuvent être des images électroniques pouvant être affichées sur un écran, simultanément ou non.

Toutes les images de l'atlas peuvent ainsi être affichées simultanément sur un écran, dans un exemple de mise en oeuvre de l'invention. En variante, une partie seulement à la fois des images de l'atlas peut être affichée sur un écran. Ainsi, par exemple, une seule image de l'atlas peut être affichée à la fois sur un écran, et l'atlas peut être configuré par exemple de telle sorte qu'une action sur un curseur permette de remplacer l'image affichée par une autre image de l'atlas, chaque image de l'atlas non affichée à l'écran pouvant être stockée, par exemple sous un format compressé, dans une mémoire électronique ou sur un support informatique tel que par exemple un disque dur ou un disque optique. Les images de l'atlas peuvent être téléchargées, par une liaison filaire ou non, depuis un serveur distant.

Les images de l'atlas peuvent être toutes des images acquises au moyen d'un appareil photographique ou d'une caméra vidéo, ou une partie au moins des images peut avoir été générée par morphage à partir d'images acquises au moyen d'une caméra vidéo ou d'un appareil photographique.

L'atlas peut comporter des boutons d'action associés respectivement aux différentes images de l'atlas et dont l'actionnement, par exemple au moyen du pointeur d'une souris d'ordinateur, permet de déclencher l'affichage de la séquence vidéo correspondante. Les boutons d'action peuvent être confondus avec les images de l'atlas.

La séquence vidéo peut être, par exemple, enregistrée sur un support informatique ou être téléchargée, par exemple après connexion à un serveur de streaming.

L'atlas peut comporter également au moins une séquence audio associée à la séquence vidéo.

L'invention a encore pour objet, selon un autre de ses aspects, un dispositif d'acquisition d'images, comportant
- au moins une caméra permettant d'acquérir une image,
- au moins un écran permettant d'afficher au moins une image acquise par la caméra, de préférence en regard d'au moins une image d'un atlas comportant plusieurs images exprimant respectivement différentes gradations d'une caractéristique de la typologie corporelle,
- des moyens de traitement permettant d'afficher sur l'écran au moins une séquence vidéo associée à l'une au moins des images de l'atlas, cette séquence comportant des images exprimant ladite caractéristique.

Les moyens de traitement peuvent comporter par exemple un micro-ordinateur, par exemple un PC portable, ou un dispositif électronique spécifique.

Les moyens de traitement peuvent comporter par exemple un micro-ordinateur présent sur le lieu d'acquisition de l'image ou distant, l'image acquise étant, dans ce dernier cas, transmise par exemple au moyen d'Internet aux moyens de traitement, la liaison pouvant être filaire ou non.

Les moyens de traitement peuvent comporter différentes unités de traitement distantes géographiquement les unes des autres.

Les moyens de traitement peuvent notamment comporter un premier micro-ordinateur sur le site d'acquisition d'une image à comparer aux images de l'atlas et au moins un deuxième ordinateur relié par une liaison informatique au premier micro-ordinateur, le traitement des données pouvant être effectué au moins partiellement par chaque ordinateur.

La caméra peut comporter un objectif grossissant, notamment d'un facteur grossissant par exemple supérieur ou égal à 20 ou 160. Pour l'observation de la peau, le facteur grossissant peut être égal par exemple à 30 environ et pour l'observation des cheveux, il peut être par exemple égal à 200 environ.

La caméra peut comporter au moins un bouton-poussoir et le dispositif peut être configuré pour figer sur l'écran une image filmée par la caméra lorsqu'une action est exercée sur le bouton-poussoir. Ainsi, l'opérateur peut par exemple promener la caméra sur la peau ou les cheveux de la personne à examiner jusqu'à trouver une zone permettant d'évaluer au plus juste l'état de sa peau ou de ses cheveux.

L'image figée à l'écran peut alors être comparée aux images de l'atlas pour choisir aisément celle qui s'en rapproche le plus.

La durée d'au moins une séquence vidéo peut être supérieure ou égale à 5 secondes, par exemple supérieure ou égale à 10 secondes, par exemple égale à 13 secondes environ.

Le dispositif peut comporter des moyens pour permettre à un observateur d'interrompre à tout moment le défilement d'une séquence vidéo. Par exemple, l'actionnement d'une touche d'un clavier ou d'un bouton d'action, par exemple tel qu'une icône, présent sur l'écran au cours du défilement de la séquence vidéo peut provoquer l'arrêt de la séquence et le retour à l'écran comportant l'image provenant de la caméra et une ou plusieurs images de l'atlas.

La caméra peut comporter un éclairage intégré.

Le dispositif peut comporter une imprimante.

Les moyens de traitement peuvent être configurés de telle sorte que les images de la séquence vidéo s'affichent avec une taille sensiblement égale à celle de l'image affichée sur l'écran provenant de la caméra.

Le dispositif peut être configuré de telle sorte que les images de la séquence s'affichent en regard d'une image provenant de la caméra, notamment une image figée.

Le dispositif peut comporter en outre un capteur apte à effectuer au moins une mesure d'une grandeur colorimétrique ou autre. Ce capteur peut par exemple être un mélanomètre, apte à mesurer la pigmentation de la peau.

Les moyens de traitement peuvent être configurés pour établir un diagnostic sur la base d'au moins une image de l'atlas sélectionnée. Des produits de traitement ou de soins peuvent alors être prescrits afin de traiter la peau ou les cheveux. Le diagnostic peut être renouvelé après un traitement ou une durée prédéterminée afin de constater une modification, par exemple une amélioration ou une détérioration de l'état de la peau ou des cheveux.

Les moyens de traitement peuvent être configurés pour envoyer à distance, par exemple à un serveur spécifique, des informations relatives à au moins une image acquise par la caméra et/ou à au moins une image de l'atlas sélectionnée.

L'invention a encore pour objet, selon un autre de ses aspects, un procédé pour évaluer une caractéristique de la typologie corporelle, dans lequel on utilise un atlas ou un dispositif d'acquisition tels que définis précédemment.

L'invention a encore pour objet, selon un autre de ses aspects, un procédé pour établir un diagnostic, par exemple cosmétique, comportant les étapes suivantes :
- permettre de sélectionner au moins une image d'un atlas parmi plusieurs images de l'atlas exprimant différentes gradations d'au moins une caractéristique de la typologie corporelle, l'image sélectionnée étant estimée correspondre à la gradation effective de la caractéristique chez une personne,
- permettre à cette personne de visualiser au moins une séquence vidéo associée à l'image sélectionnée de l'atlas, cette séquence comportant des images exprimant ladite caractéristique,
- établir un diagnostic en fonction au moins de la sélection effectuée.

Le procédé peut comporter en outre l'étape consistant à acquérir, à l'aide d'une caméra, au moins une image d'une partie de la personne exprimant la caractéristique.

Lorsque les moyens de traitement sont distants du lieu d'examen, l'image acquise par la caméra peut être transmise à distance aux moyens de traitement et le diagnostic peut également être établi à distance.

L'invention a encore pour objet, selon un autre de ses aspects, un procédé de prescription d'un produit, par exemple un produit cosmétique ou de soins, comportant les étapes suivantes :
- permettre de sélectionner au moins une image d'un atlas parmi plusieurs images de l'atlas exprimant différentes gradations d'au moins une caractéristique de la typologie corporelle, l'image sélectionnée étant estimée correspondre à la gradation effective de la caractéristique chez une personne,
- permettre à cette personne de visualiser au moins une séquence vidéo associée à l'image sélectionnée de l'atlas, cette séquence comportant des images exprimant ladite caractéristique,
- prescrire un produit, par exemple cosmétique ou de soins, en fonction au moins de la sélection effectuée.

Le procédé peut comporter en outre l'étape consistant à acquérir à l'aide d'une caméra au moins une image d'une partie du corps de la personne exprimant la caractéristique.

Dans ce cas, l'image acquise par la caméra peut être transmise à distance si les moyens de traitement sont distants du lieu d'évaluation, et la prescription du produit peut également être établie à distance.

Par « produit cosmétique » on entend un produit tel que défini dans la Directive 93/35/CEE du Conseil du 14 juin 1993, modifiant pour la sixième fois la Directive 76/768/CEE.

La caractéristique de la typologie corporelle peut d'une façon générale concerner l'état des cheveux, notamment celui du cuir chevelu ou de la fibre capillaire, ou l'état de la peau, notamment la nature de la peau ou son degré de vieillissement.

L'invention a encore pour objet, selon un autre de ses aspects, un serveur informatique configuré pour :
- permettre d'afficher au moins une image d'un atlas comportant plusieurs images correspondant à différentes gradations d'au moins une caractéristique de la typologie corporelle,
- permettre de sélectionner au moins une image de l'atlas,
- permettre d'afficher au moins une séquence vidéo associée à une image sélectionnée de l'atlas, cette séquence comportant des images exprimant ladite caractéristique.

Le serveur peut être configuré pour recevoir une image d'une partie d'une personne exprimant la caractéristique à évaluer, cette image pouvant être acquise par une caméra.

Le serveur peut également être configuré pour adresser à une adresse, par exemple une adresse électronique, un diagnostic et/ou une prescription d'un traitement et/ou des informations concernant la commercialisation de produits en rapport avec le résultat de l'évaluation.

L'invention pourra être mieux comprise à la lecture de la description détaillée qui va suivre, d'exemples de mise en oeuvre non limitatifs, et à l'examen du dessin annexé, sur lequel :
- la figure 1 est une vue schématique en perspective d'un exemple de dispositif réalisé conformément à l'invention,
- la figure 2 illustre l'utilisation du dispositif de la figure 1 pour effectuer un diagnostic capillaire,
- les figures 3 à 6 sont différents exemples de copies d'écran au cours du fonctionnement du dispositif,
- la figure 7 illustre une variante de réalisation comportant un ordinateur relié par le réseau Internet à un serveur distant, et
- la figure 8 est une copie d'écran dans une autre variante de réalisation du dispositif.

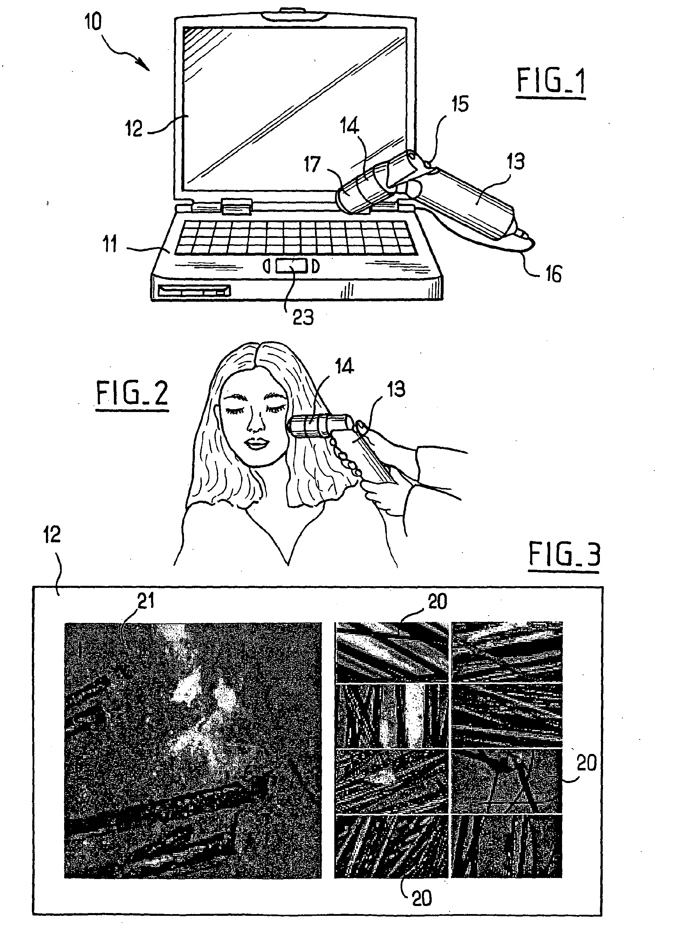

Le dispositif 10 représenté à la figure 1 comporte un micro-ordinateur 11 portable comprenant un écran 12 et une pièce à main 13 reliée au micro-ordinateur 11.

La pièce à main 13 comporte une caméra 14 munie d'un éclairage intégré et comportant au moins un bouton-poussoir 15 permettant de figer lorsque l'on appuie dessus l'image acquise par la caméra, comme cela sera détaillé plus loin. L'éclairage intégré peut être une source de lumière blanche, UV ou IR, avec possibilité éventuellement de sélectionner un type d'éclairage. L'éclairage peut être ou non polarisé. La pièce à main peut ainsi comporter, par exemple, un polariseur placé sur le trajet de la lumière émise par une source de lumière et un analyseur devant la caméra 14.

La caméra 14 peut être adaptée à effectuer une acquisition d'une image sous l'un de ces types d'éclairage.

Dans l'exemple considéré, la caméra 14 est reliée par un câble 16 au micro-ordinateur 11 mais on ne sort pas du cadre de la présente invention lorsque la pièce à main 13 est configurée pour transmettre par une liaison sans fil, par exemple radiofréquence, des informations au micro-ordinateur 11.

La caméra 14 peut être munie d'un objectif grossissant. Dans l'exemple considéré, la caméra 14 peut être munie d'un objectif de grossissement x30 ou x200 selon que l'on souhaite acquérir une image de la peau ou des cheveux.

Le micro-ordinateur 11 est configuré pour afficher à l'écran 12 des images 20 d'un atlas, ces images représentant différentes gradations d'une caractéristique de la typologie corporelle, par exemple l'état du cuir chevelu comme illustré à la figure 3.

Dans l'exemple de cette figure, l'atlas comporte huit images 20 qui correspondent à différents états du cuir chevelu : des cheveux normaux, des cheveux secs, des cheveux gras, des pellicules sèches, des pellicules grasses, la chute des cheveux chez un homme, la chute des cheveux chez une femme, et le vieillissement capillaire.

Le micro-ordinateur 11 est configuré pour afficher, en regard des images 20 de l'atlas, une image 21 provenant d'une acquisition par la caméra. Pour effectuer cette acquisition, la caméra 14 est promenée le long des cheveux, comme illustré à la figure 2, l'image observée par la caméra s'affichant en temps réel ou presque sur l'écran 12. Lorsque l'opérateur estime que l'image affichée est représentative de l'état capillaire, il appuie sur le bouton-poussoir 15, ce qui a pour conséquence de figer l'image 21 affichée à l'écran, de sorte que le dispositif d'acquisition 13 peut ensuite être posé.

L'image 21 qui est affichée à l'écran 12 est une image agrandie.

L'opérateur peut ensuite sélectionner l'une des images de l'atlas au moyen d'une souris, par exemple une souris tactile 23 du micro-ordinateur 11, en cliquant sur l'image retenue.

Le micro-ordinateur 11 est configuré de manière à provoquer l'affichage d'une séquence vidéo associée à l'image sélectionnée.

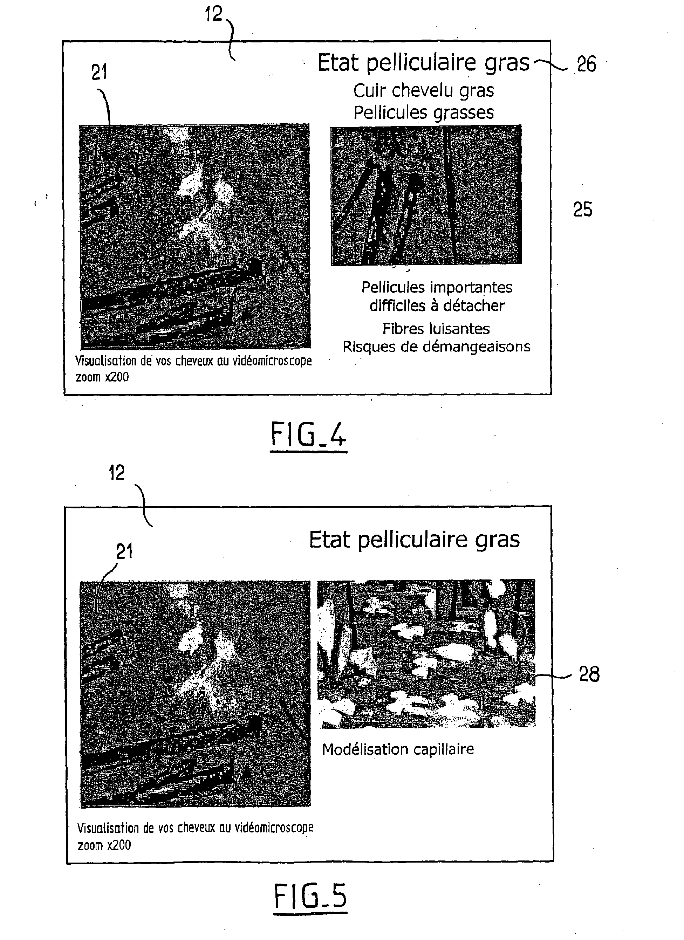

Dans l'exemple considéré, comme illustré sur la figure 4, les images 20 de l'atlas disparaissent et sont remplacées par un cadre dans lequel sont affichées successivement les images 25 de la séquence, avec le cas échéant des commentaires visuels 26 qualifiant l'état correspondant à l'image sélectionnée.

La séquence d'images 25 a été obtenue en filmant le cuir chevelu d'une personne dont l'état est estimé correspondre à celui de l'image 20 sélectionnée de l'atlas. Le cas échéant, la séquence vidéo est accompagnée d'une séquence audio, et correspond par exemple à un enregistrement sonore d'une personne commentant les images de la séquence vidéo. La séquence vidéo peut avoir une durée supérieure à 10 secondes, par exemple voisine de 13 secondes dans l'exemple considéré.

Le déroulement de la séquence peut être, dans l'exemple considéré, interrompu à tout moment par une action sur une touche du clavier du micro-ordinateur 11, par exemple. On peut ainsi arrêter la séquence sur une image donnée, ce qui permet à l'opérateur ayant effectué la comparaison entre l'image 21 à évaluer et les images de l'atlas de bien s'assurer qu'il a sélectionné la bonne image de l'atlas. L'arrêt sur image permet également à l'opérateur ayant effectué la sélection de fournir à la personne en cours d'évaluation des explications complémentaires. L'arrêt sur image permet encore, le cas échéant, de procéder à l'acquisition d'une nouvelle image 21 au moyen de la caméra 14. Ainsi, dans le cas où l'on cherche à évaluer l'état des cheveux, et que l'on procède à l'acquisition d'une image de la racine des cheveux, il peut s'avérer utile d'interrompre le défilement de la séquence lorsqu'une image de la pointe des cheveux s'affiche dans le cadre réservé aux images de la séquence, et de procéder à une nouvelle acquisition au moyen de la caméra 14 afin de mettre en évidence une similarité ou au contraire faire apparaître une différence et le cas échéant sélectionner une nouvelle image de l'atlas.

On comprend donc que si la visualisation de la séquence fait naître un doute dans l'esprit de l'opérateur sur la pertinence de son choix, l'opérateur peut sélectionner une autre image de l'atlas afin de visualiser la séquence correspondante, et après avoir visualisé les séquences associées à différentes images, décider laquelle correspond le mieux à l'état de la personne en cours d'évaluation.

La séquence d'images 25 peut se poursuivre, le cas échéant, par l'affichage d'explications, comportant par exemple des dessins 28, comme illustré sur la figure 5.

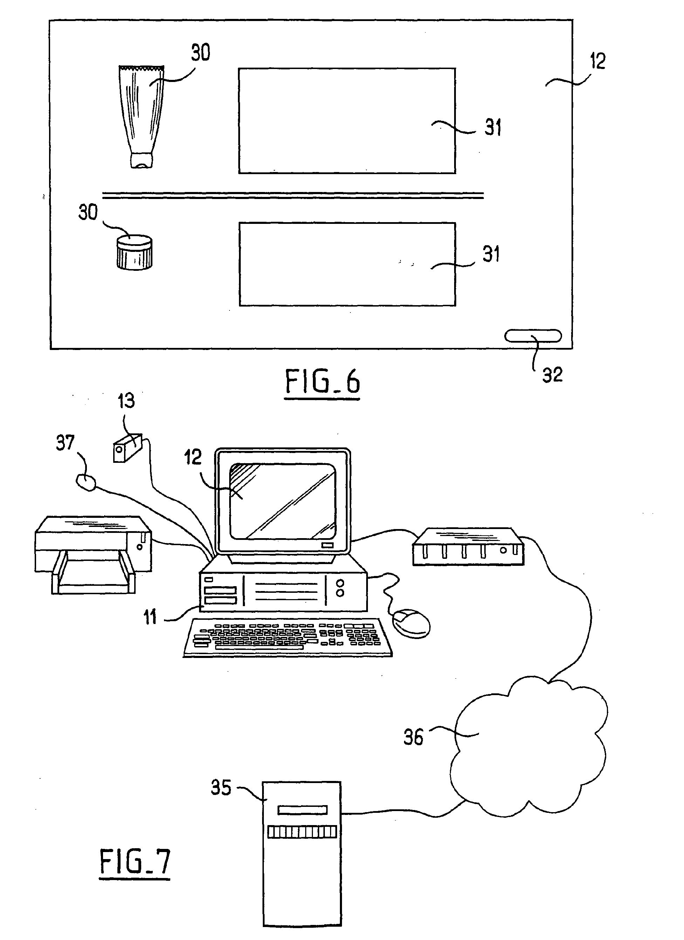

Le micro-ordinateur 11 peut être configuré pour, une fois l'évaluation terminée, effectuer un diagnostic et délivrer par exemple une prescription comme illustré à la figure 6, avec présentation à l'écran de l'image 30 de produits pouvant être utilisés pour améliorer l'état de la peau ou des cheveux de la personne évaluée, et d'un texte 31 correspondant, contenant des conseils et/ou posologie. Un bouton d'action 32 permettant de commander l'impression d'une ordonnance au moyen d'une imprimante non représentée à la figure 1 peut également être affiché, comme on le voit sur la figure 6.

Le dispositif de la figure 1 peut s'utiliser par exemple sur un point de vente ou dans un institut de beauté.

En variante, comme illustré à la figure 7, le micro-ordinateur 11, qui peut comporter une station de base, peut être relié à un serveur 35 configuré pour permettre l'affichage sur l'écran 12 du micro-ordinateur 11 des images de l'atlas. Le micro-ordinateur 11 peut être relié au serveur 35 par un réseau informatique, par exemple le réseau Internet.

Une caméra de type « Webcam », munie éventuellement d'un objectif grossissant, est reliée au micro-ordinateur 11.

On peut encore connecter éventuellement au micro-ordinateur 11 des dispositifs autres qu'une caméra vidéo, par exemple un capteur 37 configuré pour mesurer la couleur de la peau, encore appelé mélanomètre, ou d'autres types encore de capteurs, par exemple un capteur optique ou non optique destiné à déterminer un relief et/ou une propriété mécanique de la peau ou des cheveux.

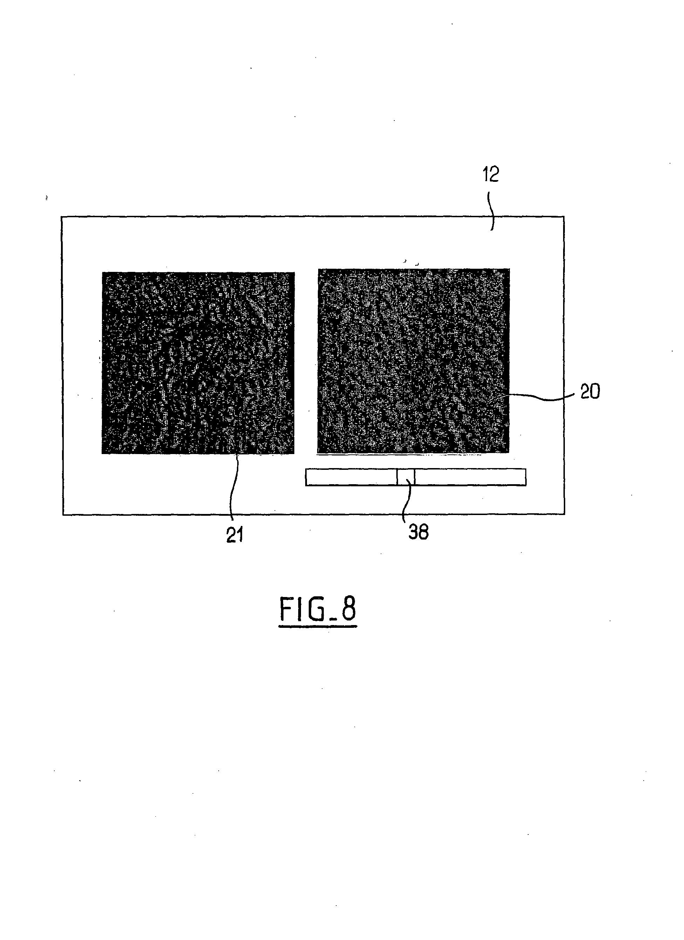

Le micro-ordinateur 11 peut être configuré pour n'afficher qu'une seule image de l'atlas à la fois, comme illustré à la figure 8.

Sur cette figure, on voit qu'une seule image de l'atlas est affichée en regard de l'image 21 acquise par la caméra.

Dans l'exemple considéré, on cherche à déterminer l'état de la peau, par exemple son degré de vieillissement. L'atlas peut comporter une pluralité d'images associées respectivement à différents degrés de vieillissement de la peau, associées à un curseur 38 qui, en étant déplacé par exemple au moyen de la souris de l'ordinateur, permet de modifier l'image affichée à l'écran.

On peut ainsi déplacer le curseur 38, par exemple de la gauche vers la droite, pour aller d'une peau jeune à une peau âgée.

Les images 20 de l'atlas qui s'affichent à l'écran peuvent être des images obtenues en photographiant ou en observant au moyen d'une caméra une image d'une peau réelle ou des images générées par morphage à partir d'au moins deux images correspondant respectivement à une peau jeune et à une peau âgée. Les images de l'atlas peuvent par exemple être générées de la manière décrite dans la demande de brevet français FR 2 818 529.

Bien entendu, l'invention n'est pas limitée aux exemples qui viennent d'être donnés.

Les images de l'atlas peuvent par exemple être des images matérielles, par exemple imprimées sur un support, et les images de la séquence être affichées sur l'écran d'un ordinateur après avoir transmis à l'ordinateur une information permettant d'identifier l'image matérielle sélectionnée.

L'image affichée à l'écran de l'ordinateur provenant de la caméra peut aussi, bien que cela soit moins avantageux, ne pas être juxtaposée aux images de l'atlas.

L'invention n'est pas limitée à l'évaluation de l'état capillaire ou cutané et s'applique également à l'évaluation du relâchement des tissus, par exemple le relâchement de la peau du cou, l'évaluation des rides, de la ptose, de la fermeté de la peau, de la cellulite.

Dans toute la description, y compris les revendications, l'expression « comportant un » doit être comprise comme étant synonyme de « comportant au moins un », sauf si le contraire est spécifié.

## Revendications

1. Atlas comportant une pluralité d'images (20) correspondant à différentes gradations d'au moins une caractéristique de la typologie corporelle et au moins une séquence vidéo (25) associée à l'une au moins des images (20) de l'atlas, cette séquence comportant des images exprimant ladite caractéristique.

2. Atlas selon la revendication 1, **caractérisé par le fait que** les images de l'atlas sont des images électroniques pouvant être affichées sur un écran (12).

3. Atlas selon l'une des revendications précédentes, **caractérisé par le fait que** toutes les images (20) de l'atlas sont affichées simultanément sur un écran (12).

4. Atlas selon l'une des revendications 1 et 2, **caractérisé par le fait qu'**une partie seulement des images (20) de l'atlas est affichée à la fois sur un écran.

5. Atlas selon l'une des revendications 1 et 2, **caractérisé par le fait qu'**au moins une image (20) de l'atlas est affichée sur un écran (12) et **par le fait que** l'atlas est configuré de telle sorte qu'une action sur un curseur (38) permette de remplacer l'image affichée par une autre image de l'atlas.

6. Atlas selon la revendication précédente, **caractérisé par le fait que** les images de l'atlas sont générées par morphage.

7. Atlas selon l'une quelconque des revendications précédentes, **caractérisé par le fait qu'**il comporte des boutons d'action associés respectivement à chaque image (20) de l'atlas et permettant de déclencher l'affichage de la séquence vidéo correspondante.

8. Atlas selon l'une quelconque des revendications précédentes, **caractérisé par le fait qu'**il comporte au moins une séquence audio associée à la séquence vidéo.

9. Dispositif d'acquisition (10) d'images, **caractérisé par le fait qu'**il comporte
- au moins une caméra (14) permettant d'acquérir une image,
- au moins un écran (12) permettant d'afficher au moins une image acquise par la caméra en regard d'au moins une image (20) d'un atlas comportant des images exprimant différentes gradations d'une caractéristique de la typologie corporelle,
- des moyens de traitement (11) permettant d'afficher sur l'écran au moins une séquence vidéo associée à l'une au moins des images de l'atlas, cette séquence comportant des images exprimant ladite caractéristique.

10. Dispositif selon la revendication 9, **caractérisé par le fait que** la caméra comporte un objectif grossissant, notamment d'un facteur grossissant supérieur ou égal à 20 ou 160.

11. Dispositif selon l'une des revendications 9 et 10, **caractérisé par le fait qu'**il comporte au moins un bouton-poussoir (15) et **par le fait que** le dispositif est configuré pour figer sur l'écran une image filmée par la caméra lorsqu'une action est exercée sur ce bouton-poussoir.

12. Dispositif selon l'une quelconque des revendications 9 à 11, **caractérisé par le fait que** les moyens de traitement comportent un micro-ordinateur portable.

13. Dispositif selon l'une quelconque des revendications 9 à 12, **caractérisé par le fait que** la durée d'au moins une séquence vidéo est supérieure ou égale à 5 secondes.

14. Dispositif selon l'une quelconque des revendications 9 à 13, **caractérisé par le fait qu'**il comporte des moyens pour permettre à un observateur d'interrompre à tout moment le défilement d'une séquence vidéo.

15. Dispositif selon la revendication 14, **caractérisé par le fait qu'**il est configuré pour permettre, après avoir interrompu le défilement d'une séquence vidéo, de procéder à une nouvelle acquisition d'une image avec la caméra, le champ d'observation de la caméra étant affiché à l'écran simultanément à l'image sur laquelle la séquence s'est arrêtée.

16. Dispositif selon l'une quelconque des revendications 9 à 15, **caractérisé par le fait que** la caméra comporte un éclairage intégré.

17. Dispositif selon la revendication 16, **caractérisé par le fait que** l'éclairage peut être sélectionné parmi les éclairages de types UV, IR ou lumière visible.

18. Dispositif selon l'une quelconque des revendications 9 à 17, **caractérisé par le fait que** les moyens de traitement (11) sont configurés de telle sorte que les images de la séquence s'affichent avec une taille sensiblement égale à celle de l'image affichée sur l'écran, provenant de la caméra.

19. Dispositif selon l'une quelconque des revendications 9 à 18, **caractérisé par le fait qu'**il est configuré de telle sorte que les images de la séquence s'affichent sur l'écran en regard d'une image, notamment un image figée, provenant de la caméra.

20. Dispositif selon l'une quelconque des revendications 9 à 19, **caractérisé par le fait qu'**il comporte un capteur apte à effectuer au moins une mesure d'une grandeur colorimétrique.

21. Dispositif selon la revendication précédente, **caractérisé par le fait qu'**il comporte un mélanomètre (37).

22. Dispositif selon l'une quelconque des revendications 9 à 21, **caractérisé par le fait qu'**il comporte une imprimante.

23. Dispositif selon l'une quelconque des revendications 9 à 22, **caractérisé par le fait que** les moyens de traitement sont configurés pour établir un diagnostic sur la base d'au moins une image de l'atlas sélectionnée.

24. Dispositif selon l'une quelconque des revendications 9 à 23, **caractérisé par le fait que** les moyens de traitement sont configurés pour envoyer à distance des informations relatives à au moins une image acquise par la caméra et/ou à au moins une image de l'atlas sélectionnée.

25. Procédé pour évaluer une caractéristique de la typologie corporelle, dans lequel on utilise un atlas tel que défini dans l'une quelconque des revendications 1 à 8 et/ou un dispositif d'acquisition tel que défini dans l'une quelconque des revendications 9 à 24.

26. Procédé pour établir un diagnostic cosmétique comportant les étapes suivantes :
- permettre de sélectionner au moins une image d'un atlas parmi plusieurs images de l'atlas exprimant différentes gradations d'au moins une caractéristique de la typologie corporelle, l'image sélectionnée étant estimée correspondre à la gradation effective de la caractéristique chez une personne,
- permettre à cette personne de visualiser au moins une séquence vidéo associée à l'image sélectionnée de l'atlas, cette séquence comportant des images exprimant ladite caractéristique,
- établir un diagnostic en fonction au moins de la sélection effectuée.

27. Procédé selon la revendication précédente, **caractérisé par le fait qu'**il comporte en outre l'étape consistant à acquérir, à l'aide d'une caméra, au moins une image d'une partie de la personne exprimant la caractéristique.

28. Procédé selon la revendication 27, **caractérisé par le fait que** l'image acquise par la caméra est transmise à distance et **par le fait que** le diagnostic est établi à distance.

29. Procédé de prescription d'un produit cosmétique, **caractérisé par le fait qu'**il comporte les étapes suivantes :
- permettre de sélectionner au moins une image d'un atlas parmi plusieurs images de l'atlas exprimant différentes gradations d'au moins une caractéristique de la typologie corporelle, l'image sélectionnée étant estimée correspondre à la gradation effective de la caractéristique chez une personne,
- permettre à cette personne de visualiser au moins une séquence vidéo associée à l'image sélectionnée de l'atlas, cette séquence comportant des images exprimant ladite caractéristique,
- prescrire un produit, notamment cosmétique ou de soins, en fonction au moins de la sélection effectuée.

30. Procédé selon la revendication précédente, **caractérisé par le fait qu'**il comporte en outre l'étape consistant à acquérir à l'aide d'une caméra au moins une image d'une partie du corps de la personne exprimant la caractéristique.

31. Procédé selon la revendication 30, **caractérisé par le fait que** l'image acquise par la caméra est transmise à distance et **par le fait que** la prescription du produit cosmétique est établie à distance.

32. Procédé selon l'une quelconque des revendications 25 à 31, **caractérisé par le fait que** la caractéristique de la typologie corporelle concerne l'état des cheveux.

33. Procédé selon l'une quelconque des revendications 25 à 31, **caractérisé par le fait que** la caractéristique de la typologie corporelle concerne l'état de la peau.

34. Procédé selon la revendication 33, **caractérisé par le fait que** la caractéristique de la typologie corporelle correspond à la nature de la peau.

35. Procédé selon la revendication 33, **caractérisé par le fait que** la caractéristique de la typologie corporelle correspond au degré de vieillissement de la peau.

36. Procédé selon la revendication 32, **caractérisé par le fait que** la caractéristique de la typologie corporelle correspond à l'état du cuir chevelu.

37. Procédé selon la revendication 32, **caractérisé par le fait que** la caractéristique de la typologie corporelle correspond à l'état de la fibre capillaire.

38. Serveur informatique, **caractérisé par le fait qu'**il est configuré pour :
- permettre d'afficher au moins une image d'un atlas comportant plusieurs images correspondant à différentes gradations d'au moins une caractéristique de la typologie corporelle,
- permettre de sélectionner au moins une image de l'atlas,
- permettre d'afficher au moins une séquence vidéo associée à une image sélectionnée de l'atlas, cette séquence comportant des images exprimant ladite caractéristique.

39. Serveur selon la revendication précédente, **caractérisé par le fait qu'**il est configuré pour recevoir une image d'une partie d'une personne exprimant la caractéristique à évaluer, cette image étant acquise par une caméra.
